**Europäisches Patentamt**

**European Patent Office**

**Office européen des brevets**

(19)

(11) Veröffentlichungsnummer : **0 456 667 B1**

(12) # EUROPÄISCHE PATENTSCHRIFT

(45) Veröffentlichungstag der Patentschrift :
21.07.93 Patentblatt 93/29

(51) Int. Cl.⁵ : **C12Q 1/02**, C12N 15/53

(21) Anmeldenummer : **90902150.3**

(22) Anmeldetag : **01.02.90**

(86) Internationale Anmeldenummer :
**PCT/DE90/00063**

(87) Internationale Veröffentlichungsnummer :
**WO 90/08836 09.08.90 Gazette 90/19**

(54) **VERFAHREN ZUM NACHWEIS VON QUECKSILBER MIT HILFE VON DURCH QUECKSILBER ZU ERHÖHTER BIOLUMINESZENZ ANGEREGTER MIKROORGANISMEN.**

(30) Priorität : **01.02.89 DE 3902982**

(43) Veröffentlichungstag der Anmeldung :
**21.11.91 Patentblatt 91/47**

(45) Bekanntmachung des Hinweises auf die
Patenterteilung :
**21.07.93 Patentblatt 93/29**

(84) Benannte Vertragsstaaten :
**AT BE CH DK ES FR GB IT LI LU NL SE**

(56) Entgegenhaltungen :
**WO-A-88/00617**
**US-A- 4 581 335**

(56) Entgegenhaltungen :
**Chemical Abstracts, Band 104, Nr. 13, 31. März
1986, (Columbus Ohio, US), D.B. Gambill et al.:
"Versatile mercuryresistant cloning and
expression vectors" siehe Seite 150, Zusammenfassung 103228a & Gene 1985, 39 (2-3),
293-7**

(73) Patentinhaber : **GENLUX
FORSCHUNGSGESELLSCHAFT FUR
BIOLOGISCHE VERFAHREN MBH
Wallstrasse 10
W-4050 Mönchengladbach 1 (DE)**

(72) Erfinder : **MÖLDERS, Horst
Zugspitzstrasse 21
W-8132 Tutzing (DE)**

(74) Vertreter : **UMLAUF, Erich O.E.
Postfach 60 08 08
W-8000 München 60 (DE)**

**EP 0 456 667 B1**

## Beschreibung

Inhalt der hier beschriebenen Erfindung ist ein Nachweisverfahren für Quecksilber-Verbindungen mit Hilfe von Mikroorganismen als Biosensoren. Der gemessene Parameter, anhand dessen sich unterschiedliche Quecksilber-Konzentrationen sensitiv und ohne Zeitverzögerung erfassen lassen, ist die Intensität des von den Organismen emittierten Biolumineszenzsignals. Grundlage der Erfindung ist die Kombination eines durch Quecksilber-Ionen induzierbaren Genkomplexes mit dem Luciferase-Gen aus Vibrio Harveyi durch die Technik molekularer Klonierung. Der resultierende Plasmidvektor, pGL4, ist so konstruiert, daß mit ihm transformierte Mikroorganismen auf erhöhte Quecksilberkonzentrationen im Wachstumsmedium mit einer erhöhten Biolumineszenz reagieren. Mit dem hier beschriebenen Plasmidsystem, pGL4, ist es möglich, verschiedenste Mikroorganismen in für Quecksilber-Ionen spezifische und empfindliche Biosensoren zu verwandeln.

### Konventionelle Meßverfahren zur Bestimmung von Schwermetall-Konzentrationen

Viele Schwermetalle sind als Spurenelemente, d.h. in geringer Konzentration, für den erfolgreichen Ablauf physiologisch wichtiger Reaktionen in lebenden Organismen von Bedeutung. In höheren Konzentrationen überwiegen bei vielen dieser Elemente allerdings toxische Effekte. Die ausgeprägte Toxizität von Quecksilber-Ionen ergibt sich aus deren Tendenz, mit Sulfhydryl-Gruppen von Cysteinen in Polypeptid-Ketten, sogenannte Mercaptide zu bilden. Dieser Effekt ist besonders nachteilig für den Organismus, wenn durch die vorgenannte Reaktion das aktive Zentrum eines Enzyms irreversibel verändert und das Enzym dadurch unwirksam wird.

Schwermetalle, wie Quecksilber, werden bislang nachgewiesen durch Methoden wie i) Potentiometrie, ii) Massenspektrometrie, iii) Atomabsorptionsspektrometrie. Bei diesen etablierten Nachweismethoden geht im allgemeinen eine höhere Genauigkeit, d.h. niedriger liegende Nachweisschwelle, einher mit höherem apparativem und zeitlichem Aufwand.

In der vorliegenden Erfindung wird nun ein Meßverfahren beschrieben, bei dem mit Hilfe von Mikroorganismen als Biosensoren Quecksilber-Ionen, spezifisch, sensitiv, mit geringem Aufwand und schnell nachgewiesen werden können.

### Stand der Technik bei der Verwendung von zur Biolumineszenz fähigen Mikroorganismen als Biosensoren

In der wissenschaftlichen - wie der Patentliteratur - ist die Verwendung von Mikroorganismen bei der Bestimmung toxischer Substanzen, z.B. in wässerigen Flüssigkeiten (US-PS 3 981 777), sowie der Bestimmung von Antibiotika-Konzentrationen (EP.No. 02000 226) beschrieben. Diesen Anwendungen liegt die Erfassung des Wachstums der Testorganismen durch klassische Methoden wie Trübungsmessungen, Auswerten der Kolonienzahl und -größe nach durchschnittlich mehr als 18 h zugrunde.

In jüngerer Zeit ist das sehr empfindliche und schnelle Verfahren der Biolumineszenz hinzugekommen; bei diesem bedient man sich natürlicherweise Biolumineszenz emittierender (PCT/ US 84/01217), oder im Labor durch Anwendung molekularbiologischer Methoden zur Biolumineszenz befähigter (Priorität vom 03.10.88, P 38 33 628.6-41) Mikroorganismen. Beiden letztgenannten Verfahren ist gemeinsam, daß durch den Vergleich relativer Absterberaten unterschiedlich sensitiver bzw. resistenter Mikroorganismen via Biolumineszenz die Erfassung genereller Toxizität in einfacher Weise möglich ist. Im Sonderfall spezifisch resistenter Organismen, deren Biolumineszenz-Signal in Anwesenheit einer Testsubstanz nicht abnimmt, besteht außerdem die Möglichkeit zur Identifikation derjenigen Substanz gegenüber welcher Resistenz vorliegt. Die Identifikation einer definierten Substanz ohne Kontroll-Messungen alleine aufgrund einer positiven Korrelation des erfaßten Meßsignals mit ansteigender Konzentration des fraglichen Toxins ist mit den vorgenannten Meßsystemen nicht möglich.

### Beschreibung der Erfindung

Inhalt der im folgenden beschriebenen Erfindung ist ein Verfahren, bei dem entsprechend ausgerüstete Mikroorganismen auf steigende Quecksilber-Konzentrationen in ihrem Umgebungsmedium mit einem erhöhten - statt wie beim Stand der Technik im Resistenzfall gleichbleibenden bzw. sich langsam abschwächenden - Biolumineszenz-Signal reagieren.

Kernstück der Entwicklung ist dabei ein mit den Methoden molekularer Klonierung geschaffener Plasmid-Vektor, in welchem Teile eines durch Quecksilber induzierbaren Operons dergestalt mit dem Luciferase (LUX)-Genkomplex aus Vibrio Harveyi verbunden sind, daß auch das für die Biolumineszenz der Mikroorganismen verantwortliche LUX-Gen der positiven Kontrolle durch Quecksilber-Ionen unterliegt. Bei diesem durch Quecksilber induzierbaren Gensystem handelt es sich um das sogenannte mer-Operon aus Plasmid R100 (Jackson, W.J. und Summers, A.O. (1982) J. Bacteriol. 149, 479-487), welches seinem Trägerorganismus die Eigenschaft der Resistenz gegenüber Quecksilberverbindungen bis zu einer Konzentration von 50 μmol/l verleiht.

Das Phänomen der Resistenz beruht auf dem Zusammenwirken dreier Polypeptide, die von einer polycistronischen Boten-RNA enkodiert werden. Nach Induktion des mer-Operons durch Quecksilber wird die Boten-RNA in verstärktem Maße gebildet, ins Cytoplasma transportiert und dort in die drei von ihr enkodierten Polypeptide translatiert: i) mer-R, ein auf die Transkription des mer-Operons insgesamt positiv wirkendes Regulator-Protein, ii) mer-TPC, ein Polypeptid, welches Quecksilber-Ionen komplexiert und ihren Transport ins Zellinnere bewerkstelligt und iii) mer-A, eine Reduktase, die (durch Übertragen zweier Elektronen) $Hg^{++}$-Ionen in die flüchtige und ungefährliche Oxidationsform $Hg°$ überführt.

Für die funktionierende Detoxifikation von Quecksilber-Ionen spielt letztlich die von mer-A enkodierte Reduktase die entscheidende Rolle: Mikroorganismen, deren mer-Operon insgesamt intakt ist, die aber eine Mutation im Quecksilber-Reduktasegen haben, sind nicht länger resistent gegenüber Quecksilber ($Hg^R$), sondern prägen einen hypersensitiven Phänotyp gegenüber Quecksilber aus ($Hg^{ss}$). Die Ursache für dieses Phänomen liegt darin begründet, daß mit Hilfe des intakten Transportproteins (mer-TPC) $Hg^{++}$-Ionen - relativ zu Mikroorganismen, die das Resistenz-Plasmid nicht tragen ($Hg^s$-Phänotyp) - in effizienter Weise in das Zellinnere translokalisiert werden, dort aber nicht reduziert und damit detoxifiziert werden.

Es ist letztlich diese Konstellation eines gegenüber Quecksilber hypersensitiv reagierenden Bakterienstammes, bei welchem anstelle des mer-A-Reduktasegens durch molekulare Klonierung der Luciferase-Genkomplex aus Vibrio Harveyi tritt, die dem im folgenden beschriebenen Verfahren zugrunde liegt.

Konstruktion eines durch Quecksilber-Ionen zu erhöhter Transkription eines Luciferase-Genkomplexes induzierten genetischen Regulationssystems: Plasmidvektor pGL4

Ausgangsplasmid bei der Klonierung eines pGL4 genannten Plasmides, das einen durch Quecksilber-Ionen transkriptionell stimulierten LUX-Genkomplex enthält, war Plasmid pDG106 (Gambill, B.D. und Summers, A.O., (1985) GENE, 39, 293-297), welches neben einem intakten, d.h. Resistenz gegen Quecksilber vermittelnden, mer-Operon zusätzlich ein Kanamycin-Resistenzgen aufweist. Aus pDG106 wurde mit Hilfe etablierter molekularbiologischer Techniken ein 2,1 Kilobasenpaare (KB) langes Restriktionsfragment (EcoR1-BamH1) entfernt, so daß ein 5,7 KB großes Vektorplasmid resultierte. Durch diese Manipulation (EcoR1-Restriktion) wird das mer-A Gen partiell deletiert, woraus - bedingt durch das Fehlen der funktionellen Quecksilber-Reduktase - der hypersensible $Hg^{ss}$-Phänotyp resultiert; die Kanamycin-Resistenz bleibt hingegen intakt und kann demzufolge zur Selektion in der anschließenden Klonierung verwendet werden.

Der in den oben beschriebenen Plasmid-Vektor zu klonierende LUX-Genkomplex aus Vibrio Harveyi wurde als 3,1 KB langes Sal1-BamH1-Restriktionsfragment, das keine Promotorsequenzen enthält, aus einem gängigen LUX-Plasmid isoliert. Um Kompatibilität der 5' - d.h. "am Anfang" - des LUX-Gens gelegenen Sal1-Schnittstelle mit der EcoR1-Schnittstelle des vorbereiteten Vektors herzustellen, bot sich eine zusätzliche Klonierung als Zwischenschritt an. Zu diesem Zweck wurde das 45 Basenpaar (BP) lange Sal1-BamH1-Restriktionsfragment aus der Polylinkerregion des kommerziell erhältlichen Klonierungsvektors pBluescript (STRATAGENE, La Jolla, CA, USA) präparativ isoliert und als Adapter-Oligonukleotid an das LUX-Fragment anligiert. Das Ligationsgemisch wird schließlich mit der Restriktionsendonuklease BamH1 nachgespalten und schließlich in einen Plasmidvektor der pBR-Familie (pUC18) kloniert. Da in dem Sal1-BamH1-Adapter-Oligonukleotid eine EcoR1-Schnittstelle vorhanden it, kann man aus dem als Zwischenstufe klonierten Plasmid nun den LUX-Genkomplex als EcoR1-BamH1-Fragment isolieren; um die Spaltung der innerhalb der LUX-Gens gelegenen zweiten EcoR1-Restriktionsstelle zu vermeiden, wurde in diesem Fall mit dem Enzym EcoR1 eine partielle Restriktionsspaltung durchgeführt, während die sich anschließende BamH1-Restriktion bis zur Vollständigkeit inkubiert wurde. Das so generierte 3,15 KB lange EcoR1-BamH1-Fragment wurde schließlich isoliert und in das oben beschriebene 5,7 KB große pDG106 Vektor-Derivat kloniert. Nach Transformation in kompetente Bakterien (E.coli, C600) zeigte sich, daß alle Kanamycin-resistenten Kolonien das gewünschte 8,85 KB-große Zielplasmid, pGL4, in stabiler Form enthielten.

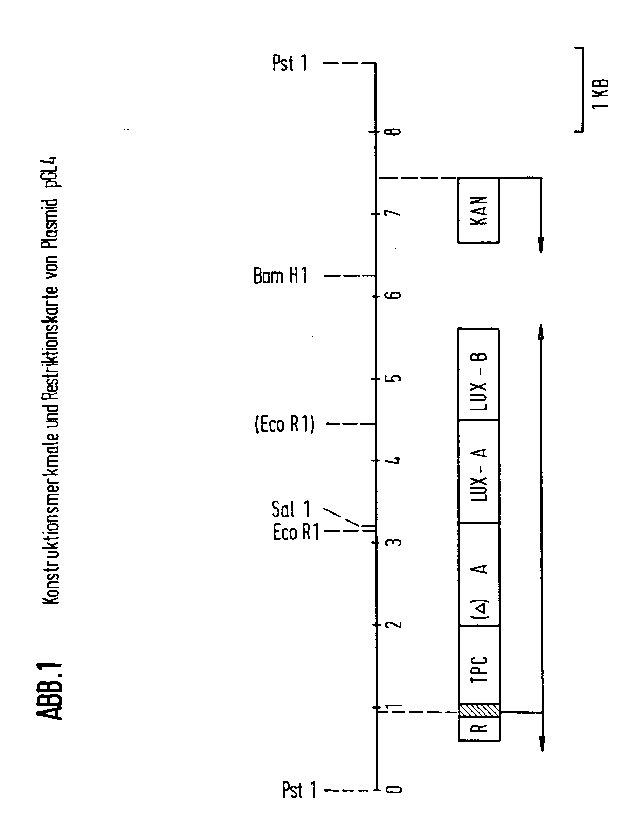

Die wesentlichen Konstruktionsmerkmale sowie eine partielle Restriktionskarte des Quecksilber-Indikatorplasmids, pGL4, sind in Abb. 1 zusammengefaßt. Das Plasmid ist in linearisierter Form (Pst1) dargestellt und hat eine Größe von 8,85 KB. Es trägt die drei Gene des mer-Operons R, TPC und A, wobei die partielle Deletion des mer-A-Reduktase-Gens als "(Δ)A" Berücksichtigung findet. Der durch Hg-Ionen induzierte Promotor des mer-Regulons ist in Höhe der 1 KB-Markierung als schraffierter Bereich dargestellt. LUX-A bzw. -B steht für die die beiden Luciferase-Untereinheiten enkodierenden Gensegmente, die ohne eigene Promotorsequenzen in das 3'-Ende des mer-A-Gens kloniert wurden.

Durch diese Konstruktionsweise wird in Plasmid pGL4, die Transkription der LUX-Gene und damit die Synthese des Luciferase-Enzymkomplexes unter die Kontrolle des mer-Operons gebracht: In Anwesenheit von Quecksilber-Ionen im Wachstumsmedium von mit pGL4 ausgestatteten Mikroorganismen wird eine erhöhte Biolumineszenzrate gemessen, die letztlich zur Identifikation und Konzentrationsabschätzung jenes Schwermetalls herangezogen werden kann.

Experimentelle Charakterisierung und Beispiele der Anwendung von mit pGL4 ausgestatteten Mikroorganismen

Zur Durchführung aller im folgenden beschriebenen Messungen wurde ein Meßgerät der Firma BERTHOLD, Biolumat LB 9500C mit folgender Einstellung benutzt:
- Meßart: Peak, - Meßzeit: 10 s, - Temperatur: 25°C, - Einstellung: manuell.

1. Nachweisempfindlichkeit des Quecksilber-Biosensors

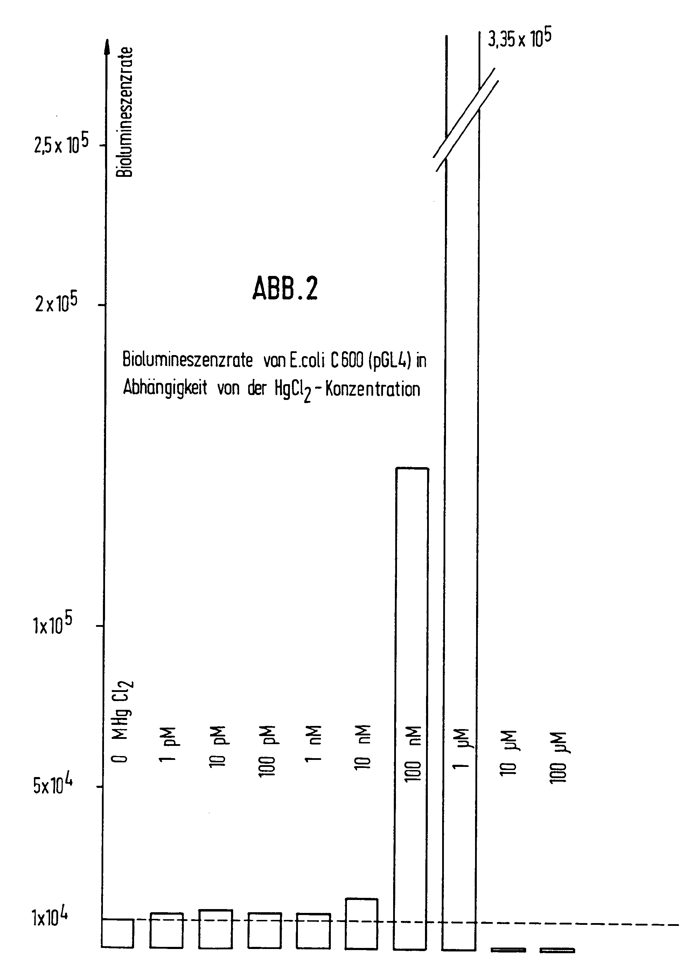

Im Hinblick auf die Nachweisempfindlichkeit von mit Plasmid pGL4 transformierten Mikroorganismen für Quecksilber wurde in einer ersten Reihe von Experimenten die Biolumineszenz-Induktion bei C600(pGL4) als Funktion der Hg-Ionen im Umgebungsmedium untersucht. Eine von einer Einzelkolonie beimpfte 1 ml-Übernachtkultur in Standard-Luria-Broth (LB)-Vollmedium unter Zusatz von 30 μg/ml Kanamycin wurde 1:10 mit sterilem LB-Medium verdünnt, um je 0,5 ml der resultierenden Bakteriensuspension mit unterschiedlichen $HgCl_2$-Konzentrationen zu induzieren. Ausgehend von 100 μmol/l wurden die Kulturen in Zehner-Verdünnungsschritten abwärts bis zu 1 pmol/l mit $HgCl_2$ versetzt und einschließlich einer Negativkontrolle zur Ermittlung des Hintergrund-Biolumineszenzsignals 35 min unter Schütteln bei 30°C inkubiert. Nach Beendigung der Inkubation wurden je 0,1 ml aus jeder Verdünnungsstufe in ein Biolumineszenzmeßgefäß überführt, mit 1 μl einer 10%igen Lösung des Luciferase-Substrates Decanal in Isopropanol versetzt und sofort mittels eines Biolumineszenzmeßgerätes (s.o.) analysiert. Die Ergebnisse - Mittelwerte aus drei voneinander unabhängigen Einzelmessungen - sind in Abb. 2 in Form eines Histogramms graphisch dargestellt. Unter den gewählten Bedingungen führt schon eine $HgCl_2$-Konzentration von 10 nmol/l relativ zur Hintergrund-Biolumineszenz (Indikatorbakterien ohne Quecksilberzusatz im Wachstumsmedium) zu einer signifikanten Erhöhung des gemessenen Signals; bei 100 nmol/l $HgCl_2$ wird eine etwa 15fache, mit 1 μmol/l eine über 30fache Steigerung der Biolumineszenzrate relativ zu nicht induzierten Kontrollkulturen erreicht. Erhöht man die Schwermetallkonzentration auf 10 μmol/l und darüber, so macht sich der gegenüber Quecksilber-Kationen hypersensible Phänotyp drastisch bemerkbar: die zu maximaler Transkription des Quecksilber-Regulons und damit zu maximaler Biolumineszenz induzierten Organismen sterben infolge der sich akut auswirkenden Toxizität des $HgCl_2$ ab.

Zur exakten Bestimmung der Nachweisempfindlichkeit des hier beschriebenen Verfahrens wurden unterhalb einer Quecksilber-Konzentration von 10 nmol/l, die - wie oben gezeigt - zu einer signifikanten Erhöhung des Biolumineszenzsignals des Biosensors relativ zum Hintergrund führt, detailliertere Messungen vorgenommen.

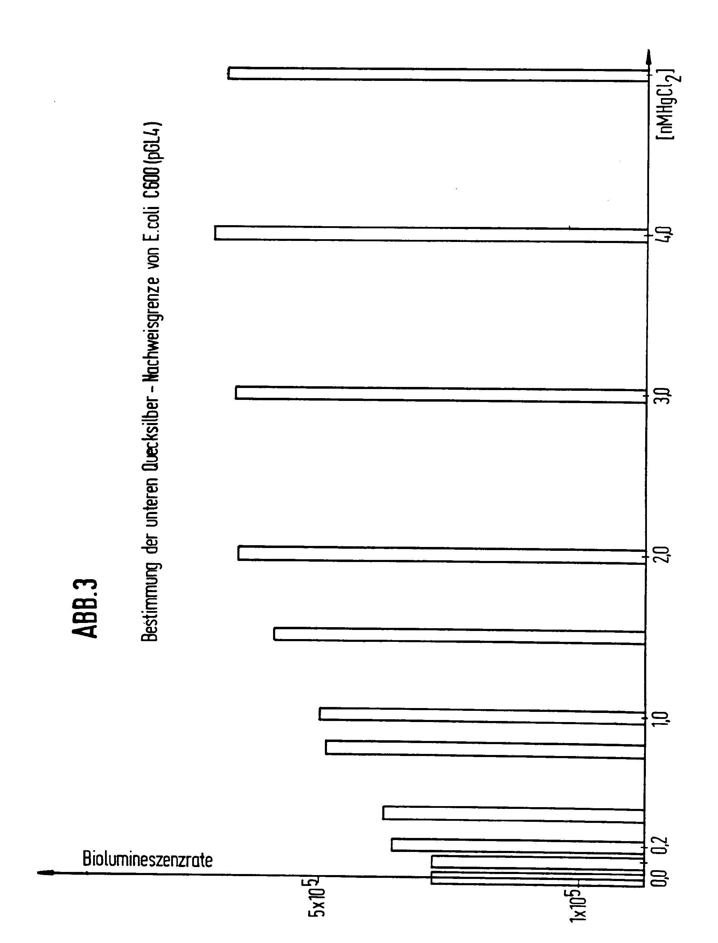

Je 0,2 ml einer 6 h bei 30°C angewachsenen, frischen Bakteriensuspension von C600(pGL4), wurden mit $HgCl_2$-Konzentrationen zwischen 0,1 und 10 nmol/l induziert. Nach 60 min Inkubation bei 37° (wie unter 2. gezeigt wird, Idealbedingungen) wurden Biolumineszenzmessungen durchgeführt, deren Ergebnisse in Abb. 3 graphisch dargestellt sind. Die Meßpunkte sind wiederum Mittelwerte aus mehreren unabhängigen Messungen, deren maximale Abweichungen 10 % vom hier dargestellten Mittelwert nicht überschreiten. Geht man vom Nullwert, d.i. die Biolumineszenzrate nicht mit $HgCl_2$-Ionen induzierter Kontrollkulturen (350 000 gemessene Lichtsignale) aus, so liegt bereits der bei 0,4 nmol/l gemessene Wert mit 400 000 außerhalb der Standardabweichung, ist also ein vom Nullwert signifikant verschiedenes Ergebnis. Dies gilt umso mehr für die um 150 000 - also 50 % des Negativkontrollwertes - von 350 000 auf 500 000 Lichtsignale erhöhte Biolumineszenz bei Einwirken von $HgCl_2$ in einer Konzentration von um 1 nmol/l.

Zusammengefaßt zeigen diese Ergebnisse, daß bereits Quecksilber-Kationen-Konzentrationen von unter 1 nmol/l (das entspricht bei einer Molekularmasse von 200,59 in wässeriger Lösung 0,2 μg/l ≙ 0,2 ppb), signifikant nachzuweisen sind. Das hier beschriebene Verfahren ist damit unter Idealbedingungen im Vergleich der absoluten Nachweisgrenzen gegenüber den am häufigsten verwendeten Methoden der Atomabsorptionsspektrometrie, deren untere Nachweisgrenzen bei etwa 2 μg/l liegt, überlegen (vgl. Tabelle I).

2. Induktionskinetik des Biolumineszenzsignals

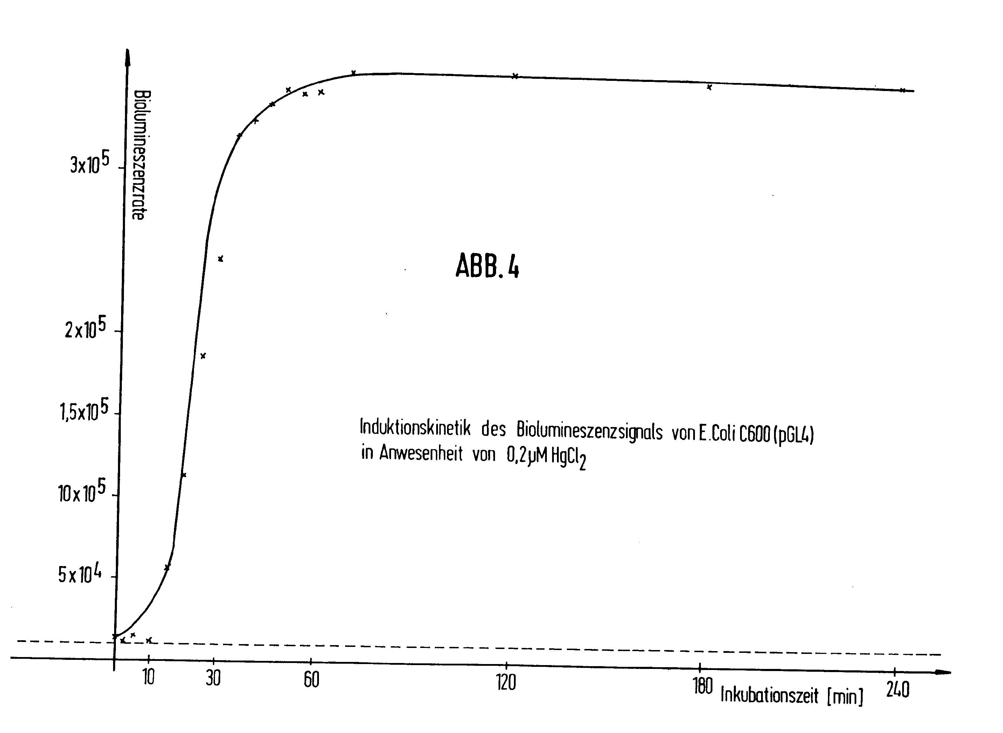

Eine wichtige Voraussetzung für meßtechnische Anwendungen des hier beschriebenen Quecksilber-Biosensors ist das Wissen um den i) zeitlichen Verlauf, ii) die maximale Signalstärke, iii) das Signal-zu-Rauschen-Verhältnis sowie iv) die Stabilität des gemessenen Signals über längere Meßzeiträume nach Induktion der Biolumineszenz. In Abb. 4 ist die Stärke der gemessenen Biolumineszenzrate als Funktion der Zeit ab dem Zeitpunkt der Zugabe von 0,2 $\mu$mol/l $HgCl_2$ - also unter Bedingungen optimaler Induktion des <u>mer</u>-Operons (vgl. Abb. 2) - aufgetragen. Nach einer etwa zehnminütigen Latenzzeit ist ein schneller Anstieg des Signals festzustellen, so daß nach 35 min 90 %, nach 50 min 100 % der maximal erreichbaren Biolumineszenzrate erreicht werden. Der Maximalwert bleibt über den Meßzeitraum von 4 Stunden konstant. Wie im unter 1. (vgl. Abb. 2) beschriebenen Experiment, beträgt das Verhältnis der schließlich erreichten maximalen Biolumineszenzrate zum Signal nicht induzierter Mikroorganismen als Referenzwert etwa 35.

Wenn auch eine direkte Linearität zwischen wirksamer $HgCl_2$-Ionen-Konzentration und gemessener Biolumineszenzrate nicht besteht, das direkte Ablesen absoluter Quecksilber-Konzentrationen mithin nicht möglich ist, so geben die Kinetik der Biolumineszenz-Induktion und der Quotient von maximal erreichtem Meßsignal und Ausgangswert zusammengenommen wichtige Informationen zur Abschätzung der $HgCl_2$-Konzentration im Umgebungsmedium der Biosensoren. Bei permanenter Aufzeichnung der Biolumineszenz, z.B. in der "online" Anwendung, sollte deshalb bei entsprechender Meßtechnik und computerunterstützter Auswertung eine exakte Konzentrationsbestimmung möglich sein.

## 3. Selektivität des Biosensors für Quecksilber-Kationen

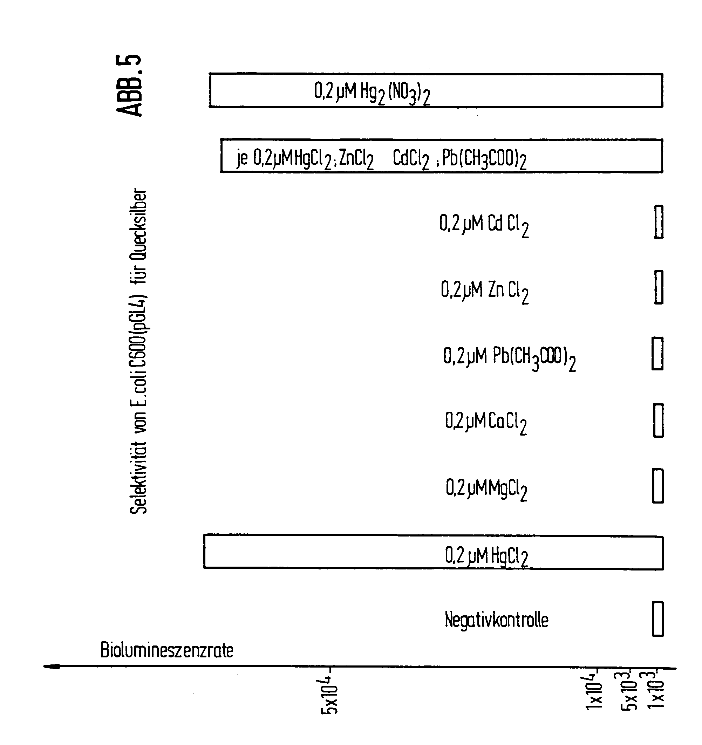

Ein wichtiges Kriterium für den Einsatz von mit pGL4 ausgestatteten Mikroorganismen als Biosensoren zur Identifikation von Quecksilber-Kationen ist deren spezifische Erkennung von Hg-Ionen. Aufgrund theoretischer Überlegungen wie empirischer, chemisch-physikalischer Untersuchungen, weisen insbesondere andere Schwermetalle in vielerlei Hinsicht Analogien zu Quecksilber auf und kommen deshalb für eine mögliche Beeinflussung der Meßergebnisse bei der Anwendung des hier beschriebenen Verfahrens in Betracht. Um die Spezifität von mit pGL4 ausgestatteten Mikroorganismen für Quecksilber-Kationen experimentell zu überprüfen, wurden je 0,5 ml C600(pGL4)-Bakterien mit 0,2 $\mu$mol/l folgender Verbindungen für 40 min unter Schütteln inkubiert: $HgCl_2$; $MgCl_2$; $CaCl_2$; $Pb(CH_3COO)_2$; $ZnCl_2$; $CdCl_2$; Gemisch aus allen vorgenannten Verbindungen; $Hg_2(NO_3)_2$. Die Auswertung der Biolumineszenzmessungen ist in Abb. 5 dargestellt.

Als Ergebnis läßt sich festhalten:

1. Sowohl $HgCl_2$ als auch $Hg_2(NO_3)_2$, also Quecksilber-Verbindungen der Oxidationsstufen +1 und +2, induzieren das Biolumineszenzsignal von C600(pGL4) in gleicher Höhe.
2. Keine der anderen getesteten Verbindungen führt zu einer Erhöhung der gemessenen Biolumineszenzrate relativ zum Hintergrund.
3. Ein Gemisch von Verbindungen, von denen einzeln nur eine ($HgCl_2$) zur Induktion des Biosensors in der Lage ist, führt zu einem Biolumineszenzsignal in gleicher Höhe wie sie die spezifisch erkannte Verbindung alleine hervorruft.

Mit pGL4 ausgerüstete Mikroorganismen sind, wie dieses Experiment zeigt, als Biosensoren für Quecksilber-Verbindungen hochspezifisch und liefern deshalb auch in Lösungen mit unbekannter Zusammensetzung zuverlässige Ergebnisse.

Anwendungsmöglichkeiten von mit pGL4 ausgestatteten Mikroorganismen

Als wesentliche Eigenschaften der hier beschriebenen Biosensoren treten hervor: i) die extrem hohe Nachweisempfindlichkeit für Quecksilber-Kationen, ii) die Selektivität der Biosensoren für Quecksilber, die auch durch Anwesenheit anderer, z.B. Schwermetall-Ionen, im Gemisch nicht beeinträchtigt wird, und iii) die Möglichkeit, ohne physikalischen Kontakt zwischen Biosensor und Signaldetektions- bzw. Verstärkungs-Einheit, damit also ohne meßtechnisch bedingte Unterbrechungen, kritische Konzentrationen von Quecksilber-Kationen nachweisen zu können. Die oben zur Charakterisierung von mit pGL4 ausgerüsteten Mikroorganismen durchgeführten Experimente stellen Beispiele für mögliche Anwendungen des Verfahrens dar. Zum einen ist der Nachweis von Quecksilber in Konzentrationen unterhalb der Detektionsgrenze, die bei etablierten Analysemethoden bestehen, bis in den Bereich <0,5 ppb möglich. In diesem Zusammenhang ist z.B. zu bedenken, daß der international zulässige Grenzwert für Quecksilber-Ionen in Trinkwasser bei 2 ppb festgelegt ist, also mit der absoluten Nachweisgrenze bei der Atomabsorptionsspektrometrie zusammenfällt. Mit dem hier beschriebenen Verfahren ist die Reevaluierung bestehender Quecksilberbelastungen, z.B. bei der Trinkwasseranalyse, ohne größeren apparativen Aufwand, also auch dezentral, möglich. Ein zweites wichtiges Anwendungsfeld ist durch die kontaktfreie Übertragungsweise des Meßsignals, Biolumineszenz, nahegelegt: Es ist möglich, Mikroorganismen, die in Durchflußbecken industrieller oder kommunaler Kläranlagen angesiedelt werden können, mit Plasmid pGL4 auszustatten. Auf diese Weise wird die Dauerüberwachung von Abwässern im Hinblick auf ihre Belastung mit Quecksilber sehr vereinfacht und gestattet, da das Meßergebnis kritische Veränderungen sofort erkennen läßt, den Aufbau eines Frühwarnsystems das "on-line" über die Abwasserqualität Auskunft geben kann.

<u>W e r t e t a b e l l e n</u>
<u>(jeweils gerundete Mittelwerte aus 3 Messungen)</u>

| <u>Zu Abb. 2</u> | <u>Konzentration $HgCl_2$</u> | <u>Biolumineszenzrate</u> |
|---|---|---|
| | 0 | 9 000 |
| | 1 pM | 10 400 |
| | 10 pM | 12 100 |
| | 100 pM | 11 800 |
| | 1 nM | 11 000 |
| | 10 nM | 16 000 |
| | 100 nM | 150 000 |
| | 1 µM | 335 000 |
| | 10 µM | 3 300 |
| | 100 µM | 50 |

| <u>Zu Abb. 3</u> | <u>Konzentration $[HgCl_2]$</u> | <u>Biolumineszenzrate</u> |
|---|---|---|
| | 0,0 | 350 200 |
| | 0,1 | 350 100 |
| | 0,2 | 386 700 |
| | 0,4 | 399 900 |
| | 0,8 | 490 600 |
| | 1,0 | 500 100 |
| | 1,5 | 570 000 |
| | 2,0 | 630 000 |
| | 3,0 | 635 000 |
| | 4,0 | 670 000 |
| | 5,0 | 650 000 |

| Zu Abb. 4 | Zeit nach Zugabe von 0,2 µM $HgCl_2$ [Minuten] | Biolumineszenzrate |
|---|---|---|
| | 0 | 11 800 |
| | 2 | 10 490 |
| | 5 | 12 064 |
| | 10 | 11 900 |
| | 15 | 57 900 |
| | 20 | 113 200 |
| | 25 | 186 600 |
| | 30 | 246 200 |
| | 35 | 321 400 |
| | 40 | 330 800 |
| | 45 | 341 800 |
| | 50 | 350 100 |
| | 55 | 348 500 |
| | 60 | 349 300 |
| | 120 | 361 000 |
| | 180 | 356 000 |
| | 240 | 357 900 |
| | 300 | 360 500 |

| Zu Abb. 5 | | Biolumineszenzrate |
|---|---|---|
| | Kein Zusatz | 1 700 |
| | 0,2 µM $HgCl_2$ | 69 000 |
| | 0,2 µM $MgCl_2$ | 1 500 |
| | 0,2 µM $CaCl_2$ | 800 |
| | 0,2 µM $Pb(CH_3COO)_2$ | 1 400 |
| | 0,2 µM $ZnCl_2$ | 1 000 |
| | 0,2 µM $CdCl_2$ | 1 100 |
| | Gemisch aus je 0,2 µM der obigen Verbindungen | 66 500 |
| | 0,2 µM $Hg_2(NO_3)_2$ | 68 000 |

T a b e l l e   I

Nachweisgrenzen Quecksilber

(jeweils Einzelelementbestimmung in schwach mineralsaurer Lösung)

| Analysenart: | | Bestimmungsgrenze: | |
|---|---|---|---|
| ICP-AES: | 10 - 20 | μg/l |
| ICP-MS: | = 0,1 | μg/l |
| Graphitrohr-AAS: | = 2 | μg/l |
| Hydrid-AAS: | = 0,5 | μg/l |
| Kaltdampf-AAS: | = 0,001 | μg/l |
| pGL4-Biosensoren: | = 0,2 | μg/l |
| (E.coli C 600) | | |

**Patentansprüche**

1. Verfahren zum nachweis von Quecksilber-Verbindungen in flüssiger oder gasförmiger Umgebung mit Hilfe von Mikroorganismen verschiedenster Herkunft und Sensitivität gegenüber toxischen Substanzen, **dadurch gekennzeichnet,** daß in die Mikroorganismen (Indikatorstämme) durch vektorvermittelten
Gentransfer ein neu konstruiertes Plasmid eingeführt wird, enthaltend
    (I) ein durch Quecksilber-Ionen spezifisch induzierbares Gen-System (mer-Operon) und
    (II) einen die Eigenschaft zur Biolumineszenz verleihenden bakteriellen Luciferase (LUX-)
    Genkomplex),
wodurch diese Mikroorganismen zu auf Biolumineszenz-Basis arbeitenden Quecksilber-spezifischen Biosensoren umgewandelt werden.

2. Verfahren nach Anspruch 1, **dadurch gekennzeichnet,** daß das neu konstruierte Plasmid folgende Konstruktionsmerkmale in sich vereint:
    (I) das Quecksilber-Regulon aus dem Quecksilber-Resistenzplasmid pDG106,
    (II) den LUX-Genkomplex aus Vibrio Harveyi sowie
    (III) ein Resistenz gegen das Antibiotikum Kanamycin vermittelndes Gen.

3. Verfahren nach Anspruch 2, **dadurch gekennzeichnet,** daß die Anwesenheit von Quecksilber-Ionen
oberhalb einer Konzentration von 0,2 μg/ℓ zur Stimulation des durch Quecksilber-Ionen induzierbaren
Promotors des Quecksilber-Regulons aus Plasmid pDG106, zu erhöhter Transkription der Gene des Operons und letztlich deren verstärkter Expression auf Proteinebene führt.

4. Verfahren nach Anspruch 3, **dadurch gekennzeichnet,** daß der LUX-Genkomplex aus Vibrio Harveyi solchermaßen durch Klonierung in das Quecksilber-Regulon eingefügt ist, daß beide Gen-Systeme gemeinsam auf einer einzigen polycistronischen Boten-RNA enkodiert werden, deren Syntheserate in direkter
Weise von der Aktivität des durch Quecksilber-Kationen induzierbaren Promotors des mer-Operons abhängig ist.

5. Verfahren nach Anspruch 4, **dadurch gekennzeichnet,** daß die durch die Anwesenheit von Quecksilber-
Kationen oberhalb einer Konzentration von 0,2 μg/ℓ vermehrt synthetisierte RNA quantitativ in die von
ihr enkodierten Proteine translatiert wird, woraus, bedingt durch das Vorhandensein erhöhter Mengen des
Enzyms Luciferase, eine erhöhte Biolumineszenz der eingesetzten Mikroorganismen resultiert.

6. Verfahren nach Anspruch 2, **dadurch gekennzeichnet,** daß die Biosensoren eine absolute Selektivität
für Quecksilber-Kationen aufweisen, so daß deren eindeutige Identifikation auch aus (Schwermetall-)Gemischen unterschiedlichster Zusammensetzung ohne weiteres möglich ist.

7. Verfahren nach Anspruch 2, **dadurch gekennzeichnet,** daß die untere Nachweisgrenze für Quecksilber-Kationen im Bereich bei 0,2 µg/$\ell$ liegt.

8. Verfahren nach Anspruch 2, **dadurch gekennzeichnet,** daß sich dieses Verfahren aufgrund seiner hohen Empfindlichkeit und der Möglichkeit, Meßwerte ohne direkten Kontakt des Biosensors mit der Signaldetektionseinheit zu erfassen, auch für on-line-Anwendungen eignet.

## Claims

1. A process for the detection of mercury compounds in liquid or gaseous environment with the aid of microorganisms of various sources and of different sensitivity towards toxic substances, **characterized in** that into the microorganisms (indicator-strains) by vector-mediated gene-transfer a newly constructed plasmid is introduced, containing
   (I) a gene-system (<u>mer</u>-Operon) specifically inducible by mercury ions
   and
   (II) a bacterial luciferase(LUX)-gene complex conferring the trait of bioluminescence,
   thereby converting these microorganisms as mercury-specific biosensors working on basis of bioluminescence.

2. A process as in claim 1, **characterized in** that the newly constructed plasmid embodies the constructional features:
   (I) the mercury regulon of the mercury-resistance plasmid pDG106,
   (II) the LUX-gene complex of Vibrio Harveyi
   and
   (III) a gene conferring resistance towards the antibiotic kanamycin.

3. A process as in claim 2, **characterized in** that the presence of mercury ions above a concentration of 0.2 µg/l leads to a stimulation of the mercury ion inducible promoter of the mercury regulon derived from plasmid pDG106, to elevated transcription of the Operon's genes and finally to their elevated expression as proteins.

4. A process as in claim 3, **characterized in** that the LUX-gene complex of Vibrio Harveyi by cloning has been positioned such within the mercury regulon, that both gene-systems are encoded commonly on a single polycistronic messenger-RNA, whose rate of synthesis is directly dependent on the activity of the <u>mer</u>-Operon-promoter being inducible by mercury cations.

5. A process as in claim 4, **characterized in** that the RNA which has been synthesized at elevated levels in the presence of mercury cations above a concentration of 0.2 µg/l is quantitatively translated into its encoded proteins, thus - caused by the presence of higher amounts of the enzyme luciferase - leading to elevated levels of bioluminescence of the microorganisms used.

6. A process as in claim 2, **characterized in** that the biosensors exhibit an absolute selectivity for mercury cations, so that their clear identification also from (heavy-metal-)mixtures of varying composition is possible without more ado.

7. A process as in claim 2, **characterized in** that the lower detection-limit of mercury cations is at a concentration of 0.2 µg/l.

8. A process as in claim 2, **characterized in** that due to its high sensitivity and the possibility of carrying out measurements without direct contact of biosensors and signal-detection unit, renders itself to be applied in permanent on-line monitoring purposes.

## Revendications

1. Procédé de détection de composés du mercure dans un environnement liquide ou gazeux, au moyen de micro-organismes d'origines les plus diverses et de sensibilité très variable, caractérisé en ce qu'on introduit dans les micro-organismes (souches indicatrices), par transfert génique à l'aide d'un vecteur, un plasmide nouvellement construit, contenant

(I) un système génique susceptible d'induction spécifique sous l'action d'ions mercure (opéron <u>mer</u>),

(II) une luciférase bactérienne (complexe LUX-gène) donnant la propriété de bioluminescence,

d'où il résulte que ces micro-organismes sont convertis en biodétecteurs spécifiques du mercure, fonctionnant sur la base de la bioluminescence.

2. Procédé selon la revendication 1, caractérisé en ce que le plasmide nouvellement construit réunit les caractéristiques de construction suivantes:

(I) le régulon au mercure, provenant du plasmide de résistance au mercure pDG106,

(II) le complexe LUX-gène provenant de Vibrio Harveyi et

(III) un gène procurant une résistance à l'antibiotique kanamycine.

3. Procédé selon la revendication 2, caractérisé en ce que la présence d'ions mercure au-dessus d'une concentration de 0,2 µg/l conduit à la stimulation du promoteur du régulon au mercure provenant du plasmide pDG106, susceptible d'induction sous l'action d'ions mercure, à une transcription accrue des gènes de l'opéron et finalement à l'expression renforcée de ceux-ci au niveau des protéines.

4. Procédé selon la revendication 3, caractérisé en ce que le complexe LUX-gène provenant de Vibrio Harveyi est inséré par clonage dans le régulon au mercure, de telle manière que les deux systèmes de gènes codent en commun pour un seul ARN messager polycistronique dont la vitesse de synthèse est directement proportionnelle à l'activité du promoteur de l'opéron <u>mer</u>, susceptible d'induction sous l'action de cations mercure.

5. Procédé selon la revendication 4, caractérisé en ce que l'ARN, dont la synthèse est accrue par la présence de cations mercure au-dessus d'une concentration de 0,2 µg/l, est traduit quantitativement en les protéines pour lesquelles il code, d'où il résulte, du fait de la présence de quantités accrues de l'enzyme luciférase, une bioluminescence accentuée des micro-organismes utilisés.

6. Procédé selon la revendication 2, caractérisé en ce que les biodétecteurs présentent une sélectivité absolue pour les cations mercure, ce qui fait que l'identification indiscutable de ceux-ci est aisément possible, même dans des mélanges (de métaux lourds) ayant les compositions les plus diverses.

7. Procédé selon la revendication 2, caractérisé en ce que la limite inférieure de détection des cations mercure est de l'ordre de 0,2 µg/l.

8. Procédé selon la revendication 2, caractérisé en ce qu'en raison de sa haute sensibilité et de la possibilité de relever des valeurs de mesure sans contact direct du biodétecteur avec l'unité de détection des signaux, ce procédé convient aussi pour les applications en ligne.

ABB.1   Konstruktionsmerkmale und Restriktionskarte von Plasmid pGL4

ABB.2

Biolumineszenzrate von E.coli C600 (pGL4) in Abhängigkeit von der $HgCl_2$ - Konzentration

ABB.3

Bestimmung der unteren Quecksilber-Nachweisgrenze von E.coli C600(pGL4)

Biolumineszenzrate

$5 \times 10^5$

$1 \times 10^5$

0,0  0,2  1,0  2,0  3,0  4,0  [nMHgCl$_2$]

EP 0 456 667 B1

ABB.4

Induktionskinetik des Biolumineszenzsignals von E.Coli C600 (pGL4) in Anwesenheit von 0,2µM HgCl₂

Axes: Biolumineszenzrate (vertical), Inkubationszeit [min] (horizontal)

EP 0 456 667 B1

# ABB. 5

Selektivität von E.coli C600(pGL4) für Quecksilber

| | Bioluminineszenzrate |
|---|---|

- $0,2\,\mu M\ Hg_2\,(NO_3)_2$
- je $0,2\mu M\,HgCl_2$ ; $ZnCl_2$    $CdCl_2$ ; $Pb(CH_3COO)_2$
- $0,2\,\mu M\ Cd\ Cl_2$
- $0,2\,\mu M\ Zn\ Cl_2$
- $0,2\,\mu M\ Pb(CH_3COO)_2$
- $0,2\,\mu M\,CaCl_2$
- $0,2\,\mu M\,MgCl_2$
- $0,2\,\mu M\ HgCl_2$
- Negativkontrolle

$5\times10^4$    $1\times10^4$  $5\times10^3$  $1\times10^3$